# EUROPEAN PATENT APPLICATION

(11) **EP 3 747 987 A1**
(43) Date of publication of application: **09.12.2020**
(21) Application number: 19178821.5
(22) Date of filing: 06.06.2019
(51) Int. Cl.: C12N 1/20, C12N 11/02

(54) **DELIVERING VIABLE MICROORGANISMS**

(71) Applicant: University of Vienna, 1010 Vienna (AT)
(72) Inventor: LIEBERZEIT, Peter, 1090 VIENNA (AT); BISMARCK, Alexander, 1090 VIENNA (AT); PERKO, Julia, 1090 VIENNA (AT); WERNER, Martin, 1090 VIENNA (AT)
(74) Representative: Dummett Copp LLP

(57) **Abstract**

A method of making polymer beads with viable microorgansims attached to the surface of the beads, for delivering viable microorganisms, the method comprising:
a) preparing an emulsion stabilised by live microorganisms; and
b) converting the emulsion to polymer beads with viable microorganisms attached to surfaces of the beads.

## Description

### BACKGROUND

### a. Field of the Invention

The present invention relates to products for delivering viable bacteria or other microorganisms to the human or animal digestive tract, and to methods of making such products.

### b. Related Art

***Fecal microbiota transplant (FMT)*** is a known therapeutic method for delivering viable and beneficial bacteria. During such therapy the stool of a healthy individual, containing fecal bacteria, is transplanted into e.g. a patient showing *Clostridium difficile* infection (CDI). FMT is described in: S. Han, S. Shannahan, and R. Pellish, "Fecal microbiota transplant: Treatment options for clostridium difficile infection in the intensive care unit," J. Intensive Care Med., vol. 31, no. 9, pp. 577-586, 2016. CDI is reported to cause several diseases, such as diarrhea, pseudomembranous sepsis, and toxic megacolon. In the worst case CDI can be fatal.

FMT is also reported as a therapy in the case of e.g. inflammatory bowel disease; P. Moayyedi, J. K. Marshall, Y. Yuan, and R. Hunt, "Canadian Association of Gastroenterology position statement: Fecal microbiota transplant therapy," Can. J. Gastroenterol. Hepatol., vol. 28, no. 2, pp. 66-68, 2014.

Currently, FMT is administered by nasojejunal tubes, colonoscopy or orally by an encapsulated product: S. D. Goldenberg et al., "Comparison of Different Strategies for Providing Fecal Microbiota Transplantation to Treat Patients with Recurrent Clostridium difficile Infection in Two English Hospitals: A Review," Infect. Dis. Ther., vol. 7, no. 1, pp. 71-86, 2018. These ways to administer the FMT ***are commonly seen as unappealing.***

J. van Wijk, T. Heunis, E. Harmzen, L. M. T. Dicks, J. Meuldijk, and B. Klumperman, "Compartmentalization of bacteria in microcapsules," Chem. Commun., vol. 50, no. 97, pp. 15427-15430, 2014, demonstrated that viable microbial cells could be encapsulated within hollow poly(organosiloxane) microcapsules by templating water-in-oil Pickering emulsion droplets via the interfacial reaction of alkylchlorosilanes. Viable cells have also previously been encapsulated in gels, including hydrogels, calcium alginate and sol-gel products. However, encapsulated bacteria may not be suitable for forming viable colonies in the gastrointestinal tract because the cells are retained within the capsule wall.

A particle stabilized or Pickering emulsion is an emulsion that is stabilized by solid particles such as silica. In principle, a broad variety of monomers or crosslinkers can be used for template immobilization via a Pickering emulsion polymerization. Polymerisation of Pickering emulsions stabilized by silica nanoparticles was performed using methacrylic acid (MAA) and ethylene glycol dimethacrylate (EGDMA) as functional monomer and crosslinking agent (Shen, X., & Ye, L. (2011). Interfacial molecular imprinting in nanoparticle-stabilized emulsions. Macromolecules, 44(14), 5631-5637). In another approach, polymerization of 2-(dimethylamino)ethyl methacrylate (DMAEMA) and EGDMA in Pickering emulsions stabilized by silica particles and Triton X - 100 was performed (Zhou, T., Kamra, T., & Ye, L. (2018). Preparation of diclofenac-imprinted polymer beads for selective molecular separation in water. Journal of Molecular Recognition, 31(3), 1-7). The work of Kujawska et al. revealed, that functional monomers 4-vinylpiridine, 2-(dimethylamino)ethyl methacrylate (DMA) and acrylamide, as well as the crosslinkers EGDMA and divinylbenzene (DVB) can be used for the polymerization of Pickering emulsions stabilized by silica nanoparticles and Triton X - 100 (Kujawska, M., Zhou, T., Trochimczuk, A. W., & Ye, L. (2018). Synthesis of naproxen - imprinted polymer using Pickering emulsion polymerization. Journal of Molecular Recognition, 31, 1-12).

The use of Pickering emulsion polymerization to synthesize polymer beads with microbial recognition sites has been reported: X. Shen et al., "Bacterial imprinting at pickering emulsion interfaces," Angew. Chemie - Int. Ed., vol. 53, no. 40, pp. 10687-10690, 2014. Bacteria were first treated with a vinyl-containing pre-polymer, N-acryl chitosan (NAC) to provide a bacterial template, and then used to stabilize an oil-in-water emulsion composed of cross-linking monomers that were dispersed in an aqueous buffer. Polymerization of the oil phase, followed by removal of the bacterial template, resulted in well-defined polymer beads bearing bacterial imprints.

The strategy of using the chitosan bacteria network to produce microspheres with bacteria immobilized onto their surface was also used Y. Cai et al (Cai, Y., Yang, S., Chen, D., Li, N., Xu, Q., Li, H., ... Lu, J. (2017). A novel strategy to immobilize bacteria on polymer particles for efficient adsorption and biodegradation of soluble organics. Nanoscale, 9(32), 11530-11536. They immobilized *Pseudomonas putina* and *Paracoccus dentrificans* on microspheres and demonstrated that the immobilized bacteria were still able to biodegrade the organic compounds phenol and DMF. Their aim was to produce material feasible for removing organic compounds from wastewater. They showed that it is possible to immobilize different kinds of bacteria. They also claimed: "Moreover, it is worth noting that this method could also be used to immobilize bacterial cells on many other functional polymer matrixes for the removal of contaminants in wastewater." However, they did not present any results which would support their statement in this paper.

L. S. Dorobantu et al. demonstrated in their work, that "intact, washed bacteria alone could stabilize oil-water emulsions by attaching to the interface, analogous to silica particles with contact angles intermediate between water-wet and oil-wet." (Dorobantu, L. S., Yeung, A. K. C., Foght, J. M., & Gray, M. R. (2004). Stabilization of Oil-Water Emulsions by Hydrophobic Bacteria. APPLIED AND ENVIRONMENTAL MICROBIOLOGY, 70(10), 6333-6336. Their experiments were based on four different bacteria strains (*P. fluorescens, R. suberifaciens, R. erythropolis, A. venetianus*), which were tested to stabilize oil in water emulsions of hexadecane/water. Their results revealed that the ability of bacteria to stabilize such a Pickering emulsion depends on the contact angle it has at the oil/water interface which depends on the hydrophilicity/hydrophobicity of the bacteria surfaces. Their work clearly demonstrates that in principle only bacteria are needed to stabilize a Pickering emulsion, although not every bacteria strain is feasible to stabilize any oil phase.

### SUMMARY OF THE INVENTION

Aspects of the invention are specified in the independent claims. Preferred features are specified in the dependent claims.

We have surprisingly found that it is possible to synthesize polymer (micro)beads with viable bacteria on their surfaces. The bacteria are firmly bound to the surfaces but can nonetheless form bacteria colonies when provided with a suitable growth medium. The invention is particularly useful for providing microbeads with viable bacteria inhabiting the human gastrointestinal tract (eg E. coli).

The polymer microbeads with viable fecal bacteria attached may be used in FMT therapy and are envisaged to provide improved patient acceptance and standardization, because they can be produced in "clean" industrial environments.

In addition to use in FMT therapy, we envisage other applications for providing beneficial substances to the human or animal digestive tract, for example for transporting probiotics to the human gastrointestinal tract.

The term "viable" is used herein to mean that the microorganism is capable of reproducing to produce a microorganism colony if provided with a suitable nutrient medium; for example a lysogeny broth (LB) medium for bacterial cultivation.

### BRIEF DESCRIPTION OF THE DRAWINGS

The invention will now be further described, by way of example only, with reference to the following drawings, in which:
Figure 1 shows chemicals used in a polymerization reaction in an embodiment of the present invention;
Figure 2 is a schematic diagram of a process in accordance with an embodiment of the invention;
Figure 3 shows a viability test of polymer beads in accordance with an embodiment of the invention, on LB-agar plates;
Figure 4 is a scanning electron microscopy image of a polymer bead with E. coli attached to its surface, in accordance with an embodiment of the invention; and
Figure 5 is a scanning electron microscopy image of E. coli attached to the bead surface of Figure 4.

### DETAILED DESCRIPTION

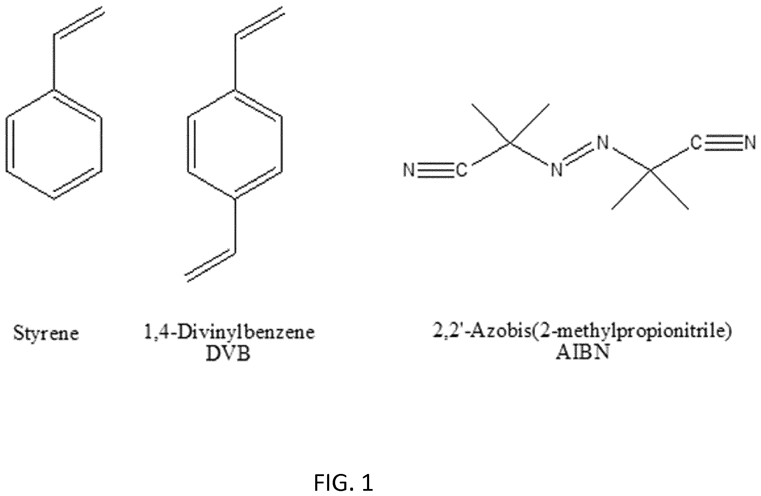

***Figure 1*** illustrates the chemicals used for the polymerization. Generally, 0.5mL Styrene (Merck KGaA, 99% purity) and 0.5mL divinylbenzene (Merck KGaA, 60% - 65% isomer ratio) were mixed together and 18mg of AIBN (Sigma Aldrich, 98% purity), which works as an initiator of the polymerization, was diluted in the monomers. The stabilizer in styrene and divinylbenzene was removed before synthesis.

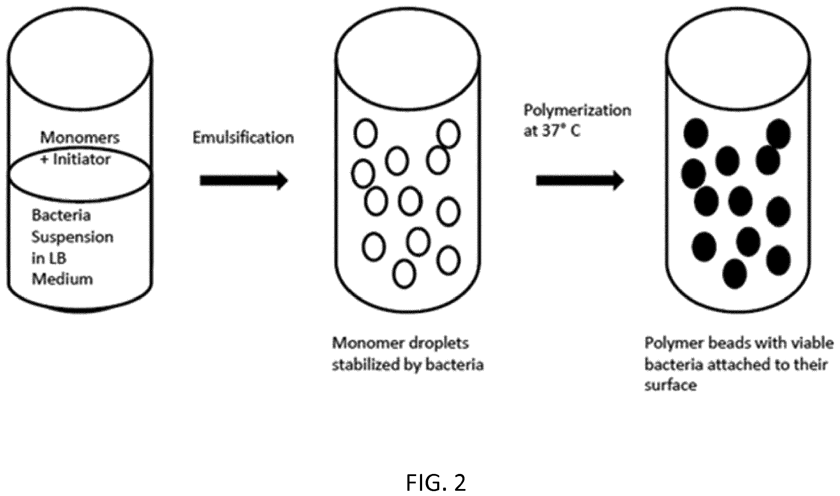

***Figure 2*** illustrates the procedure of the Pickering emulsion polymerization which was used to synthesize polymer beads with viable *E*. *coli* attached to its surface. The procedure was performed under sterile conditions. For this, the monomer solution was transferred into a sterile falcon tube. A washed pallet of cultivated *E*. *coli* W (ATCC 9637) was suspended in 4mL autoclaved LB- medium (10g/L Proteose Peptone, 5g/L NaCl, 5g/L Yeast Extract and 1g/L α-D-Glucose monohydrate). 1.2mL of the *E*. *coli* suspension was transferred to the falcon tube with the monomers. The mixture in the falcon tube contained two separate phases which could be emulsified by shaking for 30 seconds. This was a surprising result, as X. Shen *et* al had found that no stable Pickering emulsion could be obtained when the bacteria or NAC were used on their own. Afterwards, the polymerization was performed for 16,5h at 37°C. The resulting polymer beads contained viable *E*. *coli* attached to their surface.

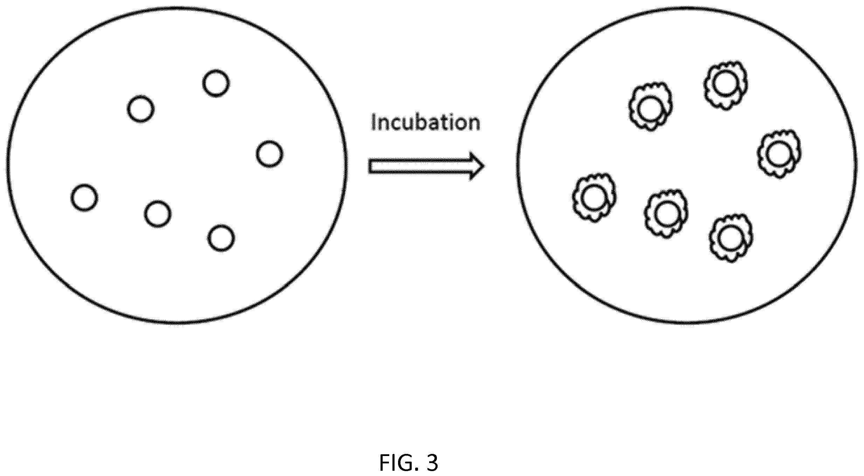

***Figure* 3** explains the viability test, which was performed to ensure, that *E*. *coli has* survived the polymerization. The viability test was performed under sterile conditions. For this, the liquid phase was removed after the polymerization and the beads were washed two times with autoclaved water. Afterwards, the beads were dried in a sterile fume hood and spread onto LB- agar plates (15g/L Agar, 10g/L Proteose Peptone, 5g/L NaCl, 5g/L Yeast Extract and 1g/L α-D-Glucose monohydrate). The plates were incubated for 48 hours at 37°C. After the incubation *E. coli* colonies were around the bead observable, which confirmed the viability of the bacteria on the polymer beads.

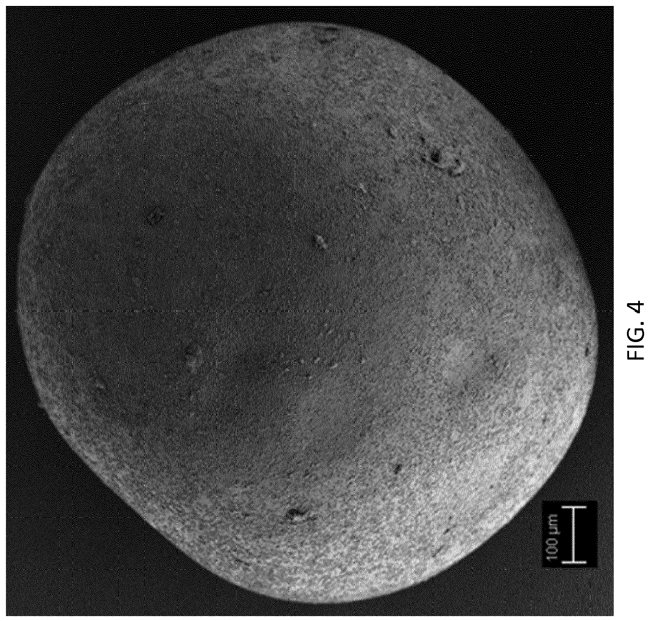

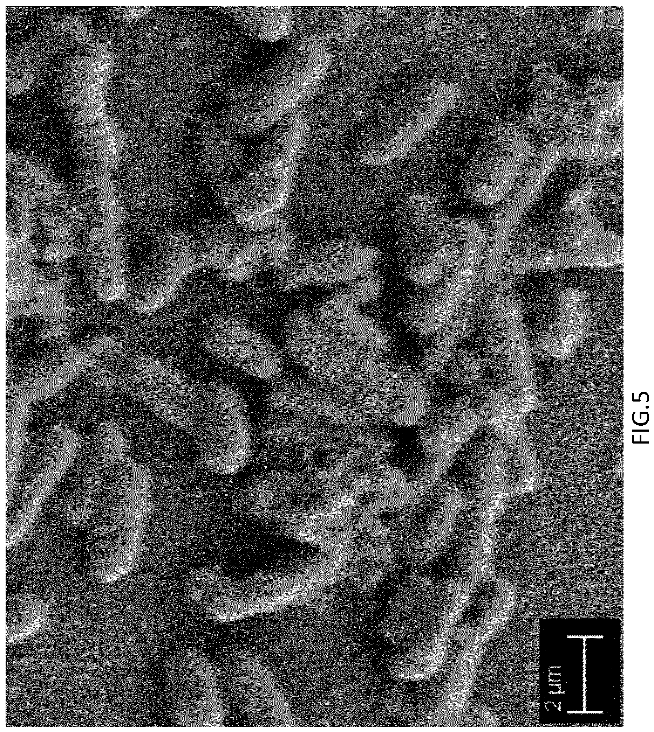

***Figure 4*** shows a scanning electron microscopy image captured from a polymer bead which was synthesized as described above. It is clearly observable, that *E*. *coli* is attached all over the surface of the beads, which ensures the loading of a large number of bacteria per bead. A closer look on the arrangement of *E*. *coli* on the surface of the beads is illustrated in ***Figure 5******.***

Although the invention has been illustrated with respect to the use of styrene monomer, it will be understood that the monomer type is not limited providing that the resulting product is physiologically acceptable to a human or animal subject. In particular, it is considered that biodegradable polymer matrices may be employed as the bead-forming material.

The invention has been illustrated with reference to thermal-initiated polymerization. However, it will be understood that the polymerization could alternatively be photo-initiated for suitable monomers, providing that the wavelength and intensity of light used does not render the bacteria or other microorganism non-viable.

However, other techniques than emulsion/suspension polymerization can be used to fabricate biodegradable polymer microspheres: One opportunity to synthesize such polymers is the solvent evaporation method. An example for biodegradable polymer spheres synthesized by this method is reported by Ito and Kawakani, who fabricated poly(lactide-co-glycolide) (PLGA) nanospheres in an oil in water emulsion via the solvent evaporation method (Ito, F., & Kawakami, H. (2015). Colloids and Surfaces A: Physicochemical and Engineering Aspects Facile technique for the preparation of monodispersed biodegradable polymer nanospheres using a solvent evaporation method. Colloids and Surfaces A: Physicochemical and Engineering Aspects, 482, 734-739). The work of Demina et al. demonstrates that the solvent evaporation method can be combined with Pickering emulsion stabilization. They synthesized poly-(L,L-lactide) microparticles via solvent evaporation in an emulsion stabilized by chitin nanocrystals (Demina, T. S., Sotnikova, Y. S., Istomin, A. V, Grandfils, C., Akopova, T. A., & Zelenetskii, A. N. (2018). Preparation of Poly (L , L-Lactide) Microparticles via Pickering Emulsions Using Chitin Nanocrystals. Advances in Materials Science and Engineering, 2018, 1-8). Without wishing to be bound by theory, we believe that immobilization of bacteria on polymer microspheres, notably biodegradable polymer microspheres, can alternatively be achieved via the combination of Pickering emulsion stabilization followed by subsequent solvent removal by evaporation.

## Claims

**1.** A method of making polymer beads with viable microorgansims attached to the surface of the beads, for delivering viable microorganisms, the method comprising:
a) preparing an emulsion stabilised by live microorganisms; and
b) converting the emulsion to polymer beads with viable microorganisms attached to surfaces of the beads.

**2.** A method according to claim 1, wherein step a) comprises:
combining an organic monomer phase and an aqueous phase containing the live microorganisms to produce a two-phase mixture;
emulsifying the two-phase mixture to produce an emulsion comprising droplets of monomer phase stabilised by live microorganisms;
and wherein step b) comprises:
polymerizing the monomer droplets to produce polymer beads with viable microorganisms attached to surfaces of the beads.

**3.** A method according to claim 1, wherein step a) comprises:
combining a solution of a polymer in an organic solvent and an aqueous phase containing the live microorganisms to produce a two-phase mixture;
emulsifying the two-phase mixture to produce an emulsion comprising droplets of solvent phase stabilised by live microorganisms;
and wherein step b) comprises:
evaporating at least some of the solvent phase to produce polymer beads with viable microorganisms attached to surfaces of the beads.

**4.** A method according to any one of the preceding claims, further comprising the steps of removing the aqueous phase and washing the beads with water.

**5.** A method according to claim 2, wherein the monomer phase comprises a mixture of styrene and a cross-linking agent, preferably 1,4- divinylbenzene.

**6.** A method according to claim 2 or claim 5, wherein the monomer phase further includes a polymerization initiator, preferably 2,2'-azo(bis)(2-methylpropionitrile) (AIBN).

**7.** A method according to any one of claims 2, 5 and 6, wherein the polymerization is carried out at a temperature in the range 35-39°C, preferably about 37°C.

**9.** A method according to any preceding claim, wherein the aqueous phase comprises the microorganism in a lysogeny broth (LB) medium.

**10.** A method according to claim 9, wherein the aqueous phase is prepared by adding the microorganism to the LB medium without pre-treatment of the microorganism with a pre-polymer.

**11.** A method according to any preceding claim, wherein the microorganism is a non-spore-forming bacterium, preferably E. coli.

**12.** A method according to claim 3, wherein the polymer is poly-(L,L-lactide).

**13.** A polymer particle with viable microorganisms attached to the surface of the particle, obtainable by the method of any one of claims 1-12.

**14.** A polymer particle according to claim 13 for use in fecal microbiota transplant (FMT) therapy.

**15.** A polymer particle according to claim 13 for use in transporting probiotics into the human or animal gastrointestinal tract.
